# EUROPEAN PATENT APPLICATION

(11) **EP 3 745 417 A1**
(43) Date of publication of application: **02.12.2020**
(21) Application number: 19305691.8
(22) Date of filing: 29.05.2019
(51) Int. Cl.: G16H 70/20

(54) **METHOD FOR CONVERTING A VIRTUAL SURGICAL PROCESS**

(71) Applicant: Université de Rennes 1, 35000 Rennes (FR); Institut National de la Santé et de la Recherche Médicale, 75013 Paris (FR)
(72) Inventor: HUAULME, Arnaud, 35000 RENNES (FR); DESPINOY, Fabien, 35000 RENNES (FR); JANNIN, Pierre, 35000 RENNES (FR); KANAKO, Harada, TOKYO (JP); MANORU, Mitsuishi, TOKYO (JP)
(74) Representative: Novagraaf Technologies

(57) **Abstract**

A method for converting a user-driven virtual surgical process performed using a simulator (1000) into a temporal sequence of semantic data describing a surgical process at a semantic level. The method comprises obtaining from the simulator (1000) at least one temporal sequence of digital data (1001) describing at a physical level the virtual surgical process simulated by the simulator in a virtual space, wherein the digital data comprise interaction data representing interactions between objects in the virtual space and kinematic data related to object displacements in the virtual space; automatically converting (1010, 1020, 1040, 1060), based on at least one configuration file (1002), said at least one temporal sequence of digital data into said temporal sequence of semantic data. The configuration file (1002) comprises semantic data representative of one or more surgical process activities and a set of one or more activity detection rules respectively associated with one or more surgical process activities defined by the semantic data.

## Description

### TECHNICAL FIELD

The disclosure generally relates to the field of surgical process modeling.

### BACKGROUND

Many computer-assisted surgical (CAS) systems have been developed in the last years. These systems have different objectives: help for the pre-operative planning, provide information and assist the surgical team in realizing a surgical process. To achieve these goals, it can be wise to use surgical process models (SPMs). A SPM is a description of a surgical process at several levels of granularity [1], for instance, phases, steps, activities. A SPM divides the surgical process into a succession of phases corresponding to the main periods of the intervention (e.g. abdominal closure). Each phase is composed of one or more steps corresponding to a surgical objective (e.g: resect the pouch of Douglas). A step is composed of a sequence of activities which describe the physical actions performed by an actor. An activity is broken down into different components, the action verb (e.g. cut), the target involved in the action (e.g. the pouch of Douglas) and the surgical instrument or tool used to perform the action (e.g. a scalpel).

SPMs could be used on multiple clinical applications such as learning and expertise assessment [3, 4], operating room optimization and management [5, 6], robotic assistance [7] and decision support [8]. An SPM could be acquired manually thanks to human observers [9]. Recent advances in machine and deep learning suggest limiting human intervention in SPM acquisition. For example, there are the automatic recognition methods for phases [10, 11], for steps [12, 13] and for activities [7, 14]. However, to train such method it is mandatory to have a data-set with a ground truth, through an own data set, or public data-sets such as JIGSAW [15], DIONE [16] or Cholec80 [11].

In deep learning, Zisimopoulos et al. [17] recently demonstrated the feasibility to train detection and classification neural network on surgical simulated data and validate it on real data. This is a hint for a track to follow that would allow to develop an automatic recognition method trained on simulated data. However, to train such method an SPMs acquisition remains mandatory. In order to obtain a completely automated SPM acquisition method, one objective would be to completely eliminate the human implication on the SPM acquisition.

There appears a need to provide a method to automatically generate surgical process models (SPMs) in an efficient and standardized manner, that avoids the complexity of a training phase by Machine Learning and / or the collection of large data sets suitable for an automatic recognition phase, and preferably that may be applied without any human implication.

### SUMMARY

According to a first aspect, there is provided a method for converting a user-driven virtual surgical process performed using a simulator into a temporal sequence of semantic data describing a surgical process at a semantic level. The method comprises:
- obtaining from the simulator at least one temporal sequence of digital data describing at a physical level the virtual surgical process simulated by the simulator in a virtual space, wherein the digital data comprise interaction data representing interactions between objects in the virtual space and kinematic data related to object displacements in the virtual space;
- automatically converting, based on at least one configuration file, said at least one temporal sequence of digital data into said temporal sequence of semantic data,
wherein the at least one configuration file comprises semantic data representative of one or more surgical process activities and a set of one or more activity detection rules respectively associated with one or more surgical process activities defined by the semantic data.

In one or more embodiments, the automatically converting comprises :
- detecting at least one surgical process activity corresponding to at least one subset of the digital data complying with at least one activity detection rule associated with the at least one surgical process activity by applying the set of one or more activity detection rules to one or more subsets of the digital data, and
- determining semantic data representative of at least one detected surgical process activity.

In one or more embodiments of the method according to the first aspect, an activity detection rule defines one or more conditions to be met by a subset of the digital data, and detecting at least one surgical process activity comprises
- filtering the interaction data to obtain one or more sequences of flag values corresponding to respective timestamps, wherein a flag value indicates whether an interaction between two objects occurs at a given timestamp;
- applying to the one or more sequences of flag values one or more activity detection rules to detect one or more pseudo-activities corresponding each to timestamp of the virtual surgical process;
- detecting for a first timestamp at least one surgical process activity based on the one or more detected pseudo-activities and contextual information, wherein the contextual information comprises filtered flag values obtained for a timestamp equal or prior to the first timestamp and / or one or more surgical process activities detected for a timestamp prior to the first timestamp.

According to a second aspect, there is provided a method for automatically generating a surgical process model. The method comprises: converting a user-driven virtual surgical process performed using a simulator into semantic data describing a surgical process at a semantic level by applying a method according to the first aspect, the semantic data describing one or more sequence of surgical process activities and / or one or more surgical process steps and / or one or more surgical process phases; and generating the surgical process model based on the semantic data.

According to a third aspect, there is provided a device comprising means for performing the steps of a method disclosed herein.

According to a fourth aspect, there is provided a computer readable medium comprising computer program instructions which when executed causes an apparatus to perform the steps of a method disclosed herein:

### BRIEF DESCRIPTION OF THE DRAWINGS

The present disclosure will be better understood and its numerous aspects and advantages will become more apparent to those skilled in the art by reference to the following drawings, in conjunction with the accompanying specification, in which:
- FIGS. 1A-1B show respectively a general and a more detailed flowchart illustrating a method for the generation of a surgical process model and corresponding method for converting a user-driven virtual surgical process performed using a simulator into a temporal sequence of semantic data describing a surgical process at a semantic level;
- FIGS. 2A and 2B show flowchart illustrating the detection of at least one surgical process activity on the basis of activity detection rules for two example embodiments;
- FIG. 3 shows a flowchart of a method for automatically generating a surgical process model;
- FIG. 4 shows a method for applying detection rules and sequentiality rules to digital data from a simulator for one specific granularity level, i.e. activities, steps, or phases;
- FIGS. 5A-5F show examples of parts of a configuration file encoding detection rules and sequentiality rules for a predefined surgical process;
- FIG. 6A show a set-up for implementing a method for converting (i.e. transcribing) a user-driven virtual surgical process and for generating a surgical process model,
- FIG. 6B show a schematic diagram showing components of a device configured to implement one or more methods described herein.
- FIGS. 7 A-7D illustrate performances of the methods for converting (i.e. transcribing) a user-driven virtual surgical process described therein.

### DETAILED DESCRIPTION

The following detailed description of certain preferred embodiments, taken in conjunction with the drawings, sets forth representative embodiments of the subject technology, wherein like reference numerals identify similar structural elements.

Examples will be described below with reference to functions, engines, block diagrams and flowchart illustrations of the method(s), system(s), device(s), apparatus(es) and computer program(s) according to the present disclosure. Each described function, engine, block of the block diagrams and flowchart illustrations may be implemented in hardware, software, firmware, middleware, microcode, or any suitable combination thereof.

In addition, it should be apparent that the teaching herein can be embodied in a wide variety of forms and that any specific structure and/or function disclosed herein is merely representative. In particular, one skilled in the art will appreciate that an embodiment disclosed herein can be implemented independently of any other embodiment and that several embodiments can be combined in various ways and that one or several aspects of different embodiments can be combined in various ways.

If implemented in software, the functions, engines, blocks of the block diagrams and/or flowchart illustrations can be implemented by computer program instructions or software code, which may be stored or transmitted over a computer-readable medium, or executed by a general purpose computer, special purpose computer or other programmable data processing apparatus to produce a machine, such that the computer program instructions or software code which execute on the computer or other programmable data processing apparatus, create the means for implementing the functions, engines, blocks described herein and / or cause the performance of the functions, engines, blocks described herein.

The automatic transcription (transcoding) of input digital data described herein is independent of any Machine Learning method. Further it does not require any step of manual annotation and can be performed in a fully automated manner.

A configuration file or a set of configuration files is used to define semantic data and a set of corresponding processing rules allowing the transcription of a pre-defined surgical process to be transcribed. The configuration file (or respectively the set of configuration files) is adapted to the pre-defined surgical process to be transcribed.

These processing rules are formulated as conditions to be met by the (filtered or non filtered) digital data collected from the virtual simulator. The content of the one or more configuration files contains all the know-how required to transcribe a set of interaction data and kinematic data into a SPM. Any type of activity (including actions e.g. of a member of a surgical team, interactions between objects, contact between objects, displacement of a tool, etc) within a SPM may be detected using such rules, especially interaction between objects. An object may here refer to a tool (e.g. a surgical tool) or any object (table, ...) used during a surgical process, a part of a body to which the surgical process applies, a part of a body of a person (e.g. hand of a surgeon or other member of a surgical team) conducting the surgical process, etc.

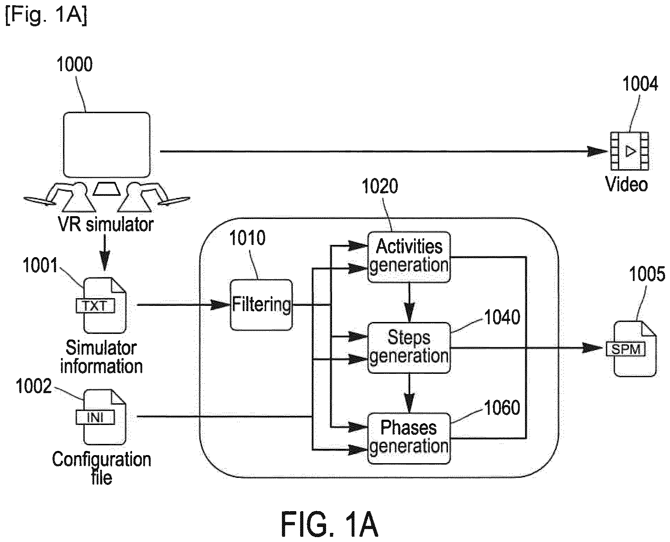

A method for converting (i.e. transcribing) a user-driven virtual surgical process performed using a simulator into a temporal sequence of semantic data describing a surgical process at a semantic level will now be described by reference to FIG. 1A.

While the different steps are described in a sequential manner, the man skilled in the art will appreciate that some steps may be omitted, combined, performed in different order and / or in parallel.

The method aims at converting output digital data provided by a simulator 1000 into a temporal sequence of semantic data describing a surgical process at a semantic level. These semantic data resulting from the conversion may further be used to generate a surgical process model (SPM), at one or more granularities levels corresponding to surgical process phases and / or surgical process steps and / or surgical process activities. Thanks to the output digital data provided by the simulator and rules described in one or more configuration files 1002, and optionally after pre-filtering 1010 of the output digital data provided by the simulator, the method allows to detect successively surgical process activities (1020), surgical process steps (1040) and surgical process phases (1060). Those three levels of semantic data granularities may be used to generate an SPM 1005.

The simulator may be a virtual simulator by means of which a 3D virtual scene simulating a surgical process is generated by a software. The simulator receives as inputs user control commands to control position and / or displacement of objects (tools, hand or surgeon, etc), forces to be applied by tools, hands, etc. The simulator generates as output a set of digital data describing the virtual scene during the simulated surgical process. The digital data describe the virtual scene on a physical level by means of physical parameters. These physical parameters include kinematic data related to object displacements in the 3D virtual space and / or interaction data interaction data representing interactions between objects in the virtual space.

The digital data may include any type of virtual scene information like: the position of each object, collision or contact between them, all kinematic information, etc. The digital data outputted by the simulator are also referred to therein as "virtual scene information" or "simulator information" 1001 as in **FIG. 1A**. Any simulator which is able to provide this kind information could be associated with the proposed method to generate SPMs. Besides, the simulator may be configured to generate videos 1004 of the performed tasks for future use of the generated SPMs as will be described in more details below.

The digital data generated by the simulator are converted into semantic data corresponding to surgical process aspects. These aspects may be defined at one or more granularity levels. In one or more embodiments, there are several granularities levels of semantic data that may be used for describing a surgical process, specifically: surgical process activities, surgical process steps and surgical process phases. A surgical process activity is a task identified by a verb (semantic data). The task may further be defined by an object target, an object tool, an actor and an effector. A surgical process step is a sequence of surgical process activities corresponding to a surgical objective (e.g.: resect the pouch of Douglas). A surgical process phase is a sequence of one or more surgical process steps.

The conversion of the digital data from the simulator into semantic data may be performed based on one or more configuration files as described herein. The processing rules used for interpreting and converting the digital data from the simulator into surgical process activities and / or surgical process steps and / or surgical process phases are defined into the one or more configuration files.

A configuration file 1002 may comprise processing rules allowing the conversion (i.e. transcription) of the digital data from the simulator into a surgical process model. The processing rules may comprise one or more activity detection rules, one or more step detection rules, one or more phase detection rules. The processing rules may further comprise one or more activity sequentiality rules, one or more step sequentiality rules, one or more phase sequentiality rules.

A configuration file 1002 may comprise a glossary / vocabulary made of semantic data representative of one or more surgical process activities and / or one or more surgical process steps and / or one or more surgical process phases.

A configuration file 1002 may further comprise the meaning and the type of each information included in the digital data from the simulator.

The first step of the method for converting a user-driven virtual surgical process performed using a simulator into a temporal sequence of semantic data is to let a user drives a virtual surgical process using a simulator 1000.

In step 1001, the digital data (simulator information) provided by the user-driven virtual surgical process performed using the simulator 1000 as defined are obtained.

In step 1010, the digital data (simulator information) may be pre-filtered, e.g. to limit artifacts due to the noise in simulator information.

In step 1020, one or more surgical process activities defined by semantic data may be generated by using the pre-filtered simulator information and the configuration file 1002 as defined previously.

In step 1040, one or more surgical process steps defined by semantic data may be generated by using the pre-filtered simulator information, the one or more surgical process activities generated in step 1020 and the configuration file 1002 as defined previously.

In step 1060, one or more surgical process phases defined by semantic data may be generated by using the pre-filtered simulator information, the one or more surgical process steps generated in step 1020 and the configuration file 1002 as defined previously.

In step 1005, a surgical process model (SPM) may be generated by using the semantic data representative of the surgical process activities, surgical process steps and surgical process phases generated in steps 1020, 1040 and 1060.

Optionally, if the simulator 1000 is able to generate videos 1004 of the tasks performed on the simulator 1000, an additional step may consist of annotating the videos 1004 generated by the simulator 1000 by using the semantic data comprised in the surgical process model generated in step 1005.

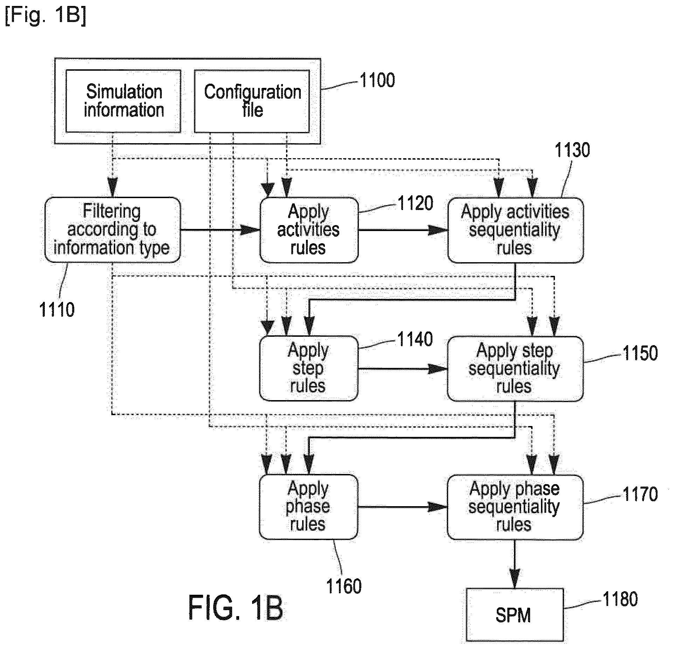

A method for converting (i.e. transcribing) a user-driven virtual surgical process performed using a simulator into a temporal sequence of semantic data describing a surgical process at a semantic level will now be described by reference to **FIG. 1B**.

While the steps are described in a sequential manner, the man skilled in the art will appreciate that some steps may be omitted, combined, performed in different order and / or in parallel.

In the algorithm presented by reference to **1B**, one or more configuration files are used at each step. A configuration file may :
- Define the type of filtering to be applied to the input data (see steps 1110);
- Define the detection rules for the different levels of granularity (steps 1120, 1140 and 1160);
- Define the sequentiality rules for the different levels of granularity (steps 1130, 1150 and 1170).

In step 1100, digital data describing at a physical level the virtual surgical process simulated by the simulator in a virtual space are obtained. The digital data may be organized in one or more temporal sequences of digital data. The digital data may comprise kinematic data related to object displacements in the virtual space and / or interaction data representing interactions between objects in the virtual space. Further in step 1100, one or more configuration files are obtained.

The conversion of the digital data from the simulator may be performed based on one or more configuration files as described herein. The processing rules used for interpreting and converting the digital data from the simulator into surgical process activities and / or surgical process steps and / or surgical process phases are defined into the one or more configuration files.

In step 1110, the digital data may be pre-filtered, e.g. to limit artifacts due to the noise in simulator information. For example, in the case of continuous data, filtering such as Kalman filtering can be used. In the case of discontinuous data, majority voting in an interval [t - N ; t + N], with N a user defined parameter can be used. For example, if N= 5, 11 values will be considered for the prefiltering.

In steps 1120 to 1180, the digital data into said temporal sequence of semantic data are automatically transcribed into a temporal sequence of semantic data describing a surgical process at a semantic level. The temporal sequence of semantic data may be included or used in the surgical process model.

A configuration file may comprise a set of one or more activity detection rules respectively associated with one or more surgical process activities defined by the semantic data. A semantic data defining a surgical process activity may be associated to one activity detection rule that enable to detect the surgical process activity in the digital data from the simulator.

In step 1120, the set of one or more activity detection rules are applied to one or more subsets of the digital data in order to detect at least one surgical process activity. At least one surgical process activity may be detected which corresponds to at least one subset of the digital data complying with at least one activity detection rule associated with the at least one surgical process activity. Semantic data representative of at least one surgical process activity detected in step 1120 are determined based on a configuration file defining such semantic data.

A configuration file may further comprise a set of one or more activity sequentiality rules. An activity sequentiality rule enables to detect a predetermined sub-sequence of surgical process activities in a sequence of one or more detected surgical process activities.

In step 1130, the set of one or more activity sequentiality rules are applied to one or more subsets of detected surgical process activities in order to detect at least one sequence of activities. In step 1130, at least one sequence of activities are detected.

A configuration file may comprise semantic data representative of one or more surgical process steps. A configuration file may comprise a set of one or more step detection rules respectively associated with one or more surgical process steps defined by the semantic data. A semantic data defining a surgical process step may be associated to one procedure step detection rule that enable to detect the surgical process step in the digital data from the simulator.

A surgical process step may correspond to a sequence of surgical process activities occurring between a starting point and an ending point.

In step 1140, the set of one or more step detection rules to one or more subsets of the digital data and / or to one or more sequence of surgical process activities detected in step 1130 in order to detect at least one surgical process step. In step 1140, at least one surgical process step is detected which corresponds to a subset of at least one detected surgical process activity complying with at least one step detection rule associated with the at least one surgical process step. Semantic data representative of at least one detected surgical process step detected in step 1140 are determined based on a configuration file defining such semantic data.

A configuration file may comprise a set of one or more step sequentiality rules. A step sequentiality rule enables to detect a predetermined sub-sequence of surgical process steps in a sequence of one or more detected procedure steps.

In step 1150, the set of one or more step sequentiality rules are applied to one or more subsets of detected surgical process steps to detect at least one sequence of surgical process steps. In step 1150, at least one sequence of surgical process steps is detected.

A configuration file may comprise semantic data representative of one or more surgical process phases. A configuration file may comprise a set of one or more phase detection rules respectively associated with one or more surgical process phases defined by the semantic data. A semantic data defining a surgical process phase may be associated to one procedure phase detection rule that enable to detect the surgical process phase in the digital data from the simulator.

A surgical process phase may correspond to a sequence of surgical process steps occurring between a starting point and an ending point.

In step 1160, the set of one or more phase detection rules are applied to one or more subsets of the digital data and / or to one or more sequence of surgical process steps detected in step 1150 in order to detect at least one surgical process phase. In step 1160, at least one surgical process phase may be detected which corresponds to a subset of one or more detected surgical process steps complying with at least one phase detection rule associated with the at least one surgical process phase. Semantic data representative of at least one surgical process step detected in step 1160 are determined based on a configuration file defining such semantic data.

A configuration file may comprise a set of one or more phase sequentiality rules. A phase sequentiality rule enables to detect a predetermined sub-sequence of surgical process phases in a sequence of one or more detected procedure phases.

In step 1170, the one or more phase sequentiality rules are applied to one or more subsets of detected surgical process steps to detect. In step 1170, at least one sequence of surgical process phases is detected. Semantic data representative of the detected at least one sequence of surgical process phases detected in step 1170 are determined based on a configuration file defining such semantic data.

In step 1180, a temporal sequence of semantic data describing a surgical process at a semantic level is generated from the semantic data defined in the configuration file.

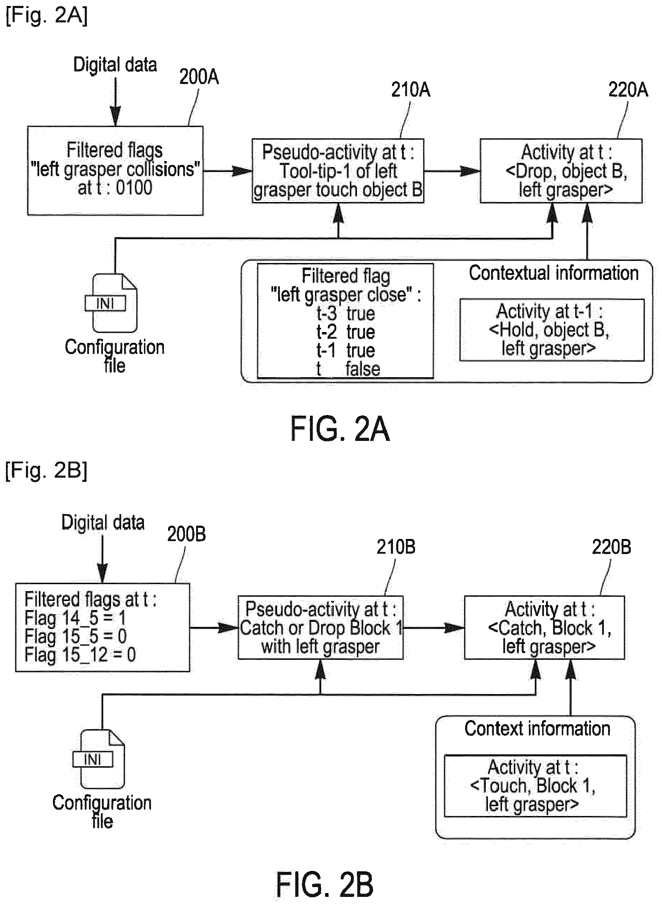

A method for detecting at least one surgical process activity from digital data describing at a physical level a virtual surgical process simulated by a simulator in a virtual space will now be described by reference to **FIGS. 2A-2B**, which illustrates two different examples of surgical process activity detection.

While the steps are described in a sequential manner, the man skilled in the art will appreciate that some steps may be omitted, combined, performed in different order and / or in parallel. The method may be used to implement step 1120 of a method for transcribing a user-driven virtual surgical process described by reference to **FIG. 1B**.

In steps 200A (respectively 200B) in FIGS. 2A-B, digital data from the simulator are converted to binary data, that is to one or more sequences of flag values. This enables to work on binary data rather on the raw data from the simulator therefore simplifying the later detection of activity, step or phase. Alternatively, the activity detection rules may be applied directly to the raw (i.e. non binary) digital data from the simulator..

One or more sequences of flag values is / are computed from the digital data such that a flag value correspond to a timestamp. A flag value indicates whether the digital data - or more specifically, the interaction data included in the digital data - fulfill or not at a given timestamp one or more conditions. A condition may be that a given event occurs, thus a flag value indicates whether the event occurs or not at a given timestamp. A condition may be for example that an interaction (e.g contact or a collision) between two objects occurs at a given timestamp. More precisely, a flag value indicates whether an interaction between two objects is represented at a given timestamp by the interaction data. The interaction data may already be binary data. The interaction data are filtered to obtain the one or more sequences of flag values, e.g. in order to limit artifacts due to the noise in the digital data from the simulator. A flag value indicates whether an interaction between two objects occurs at timestamp t.

In the case of step 200A of FIG. 2A, the flag values of interest indicates whether the left grasper collision occurs at a time stamp t, and distinct flag values are obtained for respective distinct objects with which the collision is determined. In the case of step 200B of FIG. 2B, other specific conditions elements are checked using the flag values.

In steps 210A-210B, the surgical activity detection rule(s) of the configuration file is(are) used to define how to interpret the sequence of flags values as a pseudo-activity. A pseudo-activity may thus be detected for each interaction or collision detected by means of the flag values. A pseudo-activity corresponds to a time period of the virtual surgical process. A pseudo-activity is a surgical process activity which only takes into account the interaction between objects without any context information. A surgical activity detection rule is applied to the sequence of flags values to determine the pseudo-activity corresponding to the detected interaction / collision and the concerned objects. For example, in the case of FIG. 2A, step 210A, the interpreted pseudo-activity may be semantically formulated as "tool-tip-1 of left grasper touches object B". In the case of FIG. 2B, step 210B, the interpreted pseudo-activity may be semantically formulated as "left grasper catches or drops block 1".

In steps 220A, 220B in **FIGS. 2A-2B**, the current corresponding surgical process activity can be deduced by adding context information.

In steps 220A, 220B in **FIGS. 2A-2B**, in the block named "activity at t", a surgical process activity corresponding to the previously detected pseudo-activity is detected from one or more detected pseudo-activities for a first time period. In one or more embodiments, contextual information may be used for the detection of at least one surgical process activity. The contextual information may comprise filtered flag values obtained for a time period prior to the first time period. The contextual information may comprise one or more surgical process activities detected for a time period prior to the first time period. For a given timestamp, the contextual information may include an activity detected at one or more previous timestamps t-1, t-2, etc and / or a sequence of flag values, obtained at step 200A or 200B, at the current timestamp t and / or at one or more previous timestamps t-1, t-2, t-3.

In the example of step 220A of FIG. 2A, the contextual information includes both the filtered flag values relative to the closure of the left grasper at timestamps t-3, t-2, t-1 and t and the activity detected at timestamp t-1. A modification of the closure flag from true at t-1 to false at t can be observed. Besides, the previous activity at t-1 consists of holding the object B, i.e. both tool-tips were in contact with this object at t-1. From this information, that is, opening of the grasper, and having previously two contacts with object B and now only one, the current activity "left grasper drops the object B" may be detected.

In the example of step 220B of FIG. 2B, specific conditions elements are checked using the flag values. The configuration file may then interpret those flags as the pseudo-activity previously mentioned ("left grasper catches or drops block 1"), as shown by the arrow going from the configuration file icon to the "pseudo-activity at t" block.. In this case, the contextual information may comprise the surgical process activity detected for a time period prior to the first time period, consisting in touching the block 1 with the left grasper. From this contextual information, the current activity 'left grasper catches the block 1" may be detected.

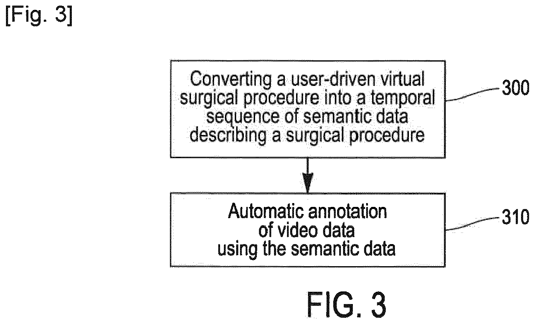

A method for automatically generating a surgical process model will now be described by reference to **FIG. 3**. While the steps are described in a sequential manner, the man skilled in the art will appreciate that some steps may be omitted, combined, performed in different order and / or in parallel.

In step 300, a user-driven virtual surgical process performed using a simulator is transcribed into semantic data describing a surgical process at a semantic level by applying a method for converting a user-driven virtual surgical process according to any embodiment described herein. The semantic data describing the surgical process may be semantic data describing one or more sequence of surgical process activities and / or one or more surgical process steps and / or one or more surgical process phases. The surgical process model is generated based on the semantic data.

In one or more embodiments, the surgical process model includes an annotated video. In step 310, video data are automatically annotated by using the semantic data to generate an annotated video as part of the surgical process model.

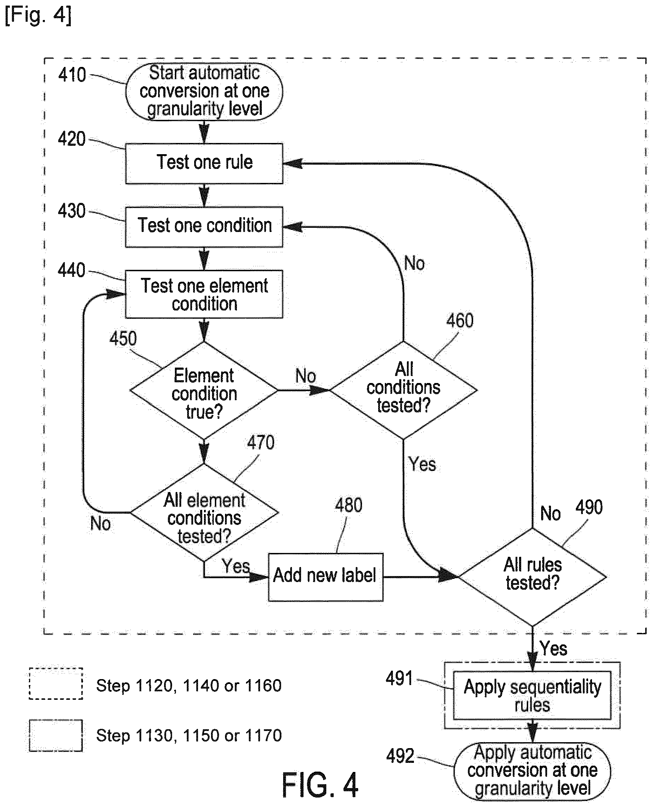

A method for applying detection rules and sequentiality rules to digital data from a simulator will now be described by reference to **FIG. 4**.

**FIG. 4** shows in detail how the rules are tested for a given level of granularity (activity, step or phase). All detection rules are checked before applying the sequentiality rules to the result of the detection rules. This is due to the fact that for each level of granularity, several actors can be at the origin of the activities, or the activities may concern different objects. Thus, for example, it is possible to take object 1 and object 2 with the right hand (actor1) while the left hand (actor 2) cuts object 3. So in this example 3 rules may be defined and checked:
- Take object 1 with actor 1;
- Take object 2 with actor 1;
- Cut object 3 with actor 2.

Instead of defining 3 rules for this particular case, it would also be possible to define only one rule that combines the conditions of these 3 rules.

This method described by reference to **FIG. 4** may be used to implement steps 1120 and 1130 on a first granularity level, corresponding to the activity level, of a method for transcribing a user-driven virtual surgical process described by reference to **FIG. 1B** or **FIG. 1A**. This method may similarly be used to implement steps 1140 and 1150 on a second granularity level corresponding to the surgical process step level. This method may similarly be used to implement steps 1160 and 1170 on a third granularity level corresponding to the surgical process phase level.

While the steps are described in a sequential manner, the man skilled in the art will appreciate that some steps may be omitted, combined, performed in different order and / or in parallel.

In step 410, the transcription (conversion) is started at one granularity level. The concerned rules are loaded: the rules include a set of detection rules and a set of sequentiality rules. For example, the activity detection rule and activity sequentiality rules are loaded for the first granularity level. For example, the step detection rules and step sequentiality rules are loaded for the second granularity level. For example, the phase detection rules and phase sequentiality rules are loaded for the third granularity level. For clarity purposes, only the processing at the first granularity level is described. But the description can be transposed without difficulty to the other granularity levels.

In step 420, a first detection rule of the set of detection rules is selected. The first detection rule defines one or more conditions to be met by a first subset of digital data from the simulator. The first detection rule is the current detection rule for the execution of the following steps 430-480.

In step 430, a first condition of the one or more conditions of the current detection rule is selected. The first condition is the current condition for the execution of the following steps 440-470.

In step 440, a first element of the current condition is selected. The first element is the current element for the execution of the following step 450..

In step 450, the current element is applied to a first subset of digital data to determine whether the current element is verified by the first subset of digital data. If the current element is verified by the first subset of digital data, step 470 is executed after step 450. If the current element is not verified by the first subset of digital data, step 460 is executed after step 450.

In step 460, it is determined whether all conditions of the current detection rule have been verified on the first subset of digital data. If all conditions of the current detection rule have been verified on the first subset of digital data, step 490 is executed after step 460. If at least one condition of the current detection rule has not been verified on the first subset of digital data, step 430 is executed again after step 460. For this next execution of step 430, another current condition will be selected among the one or more conditions of the current detection rule selected in the previous execution of step 420.

In step 470, it is determined whether all elements of the current condition have been tested on the first subset of digital data. If all elements of the current condition have been tested on the first subset of digital data, then step 480 is executed after step 470. If at least one element of the current condition has not been tested on the first subset of digital data, then step 440 is executed again after step 470. For this next execution of step 440, another current element will be selected among the one or more element of the current condition selected in the previous execution of step 430.

In step 480, all elements of at least one condition of a current rule have been verified on the first subset of digital data. Therefore a surgical process activity is detected for the first subset of digital data. The semantic data associated with the current rule are extracted from the current file and a label or semantic data defining an activity are assigned to the first subset of digital data.

In step 490, it is determined whether all detection rules of the set of detection rule have been tested. If all detection rules of the set of detection rule have been tested, the step 491 is executed after step 490. If at least one detection rule of the set of detection rule has not been tested, the step 420 is executed again after step 490. For this next execution of step 420, another current rule will be selected among the set of detection of the concerned granularity level.

In step 491, the sequentiality rules are applied to the temporal sequence of surgical process activities detected in the different executions of step 480. For each activity sequentiality rule, it is determined whether a predetermined sequence of surgical process activities is present in this temporal sequence of surgical process activities.

When step 491 is executed for the last granularity level, i.e. by applying phase sequentiality rule, the result of the execution of step 491 enables to identify a predetermined sequence of surgical process phases, and thus to transcribe the surgical process corresponding to the one or more configuration files used as input. For example, a digestive procedure, or an arthroscopic procedure, may be transcribed and for each surgical process one or more corresponding configuration files are used.

In step 492, the automatic conversion performed at a given granularity level ends.

The digital data are split into subsets of digital data, where one subset correspond to a timestamp t. The steps 410-492 may thus be repeated for each timestamp t and the corresponding subset of digital data.

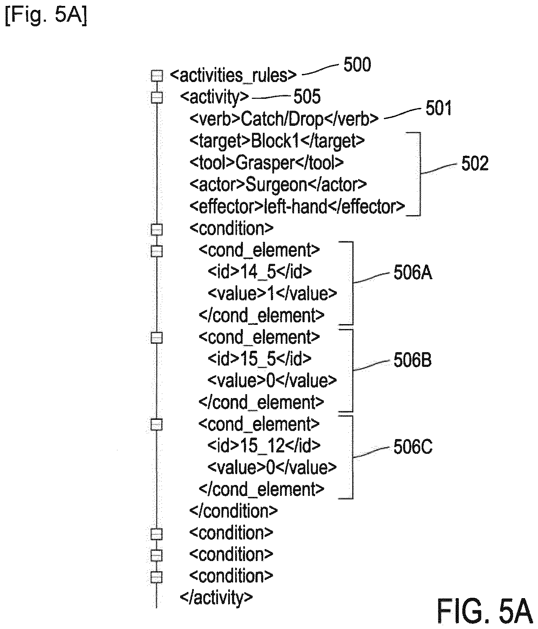

FIGS. 5A-5F show examples of parts of a configuration file encoding detection rules and sequentiality rules for a pre-defined surgical process. A configuration file may be an XML file or may use any encoding language for encoding one or more conditions to be tested on digital data. An XML has several advantages, the main one being that it can be easily understood both by the machine and by humans. However, other file types could be used such as text files or binary files. In XML files, a data field is identified by a named tag.

As this will be illustrated by the description of FIGS. 5A-5F, each processing rule is defined by reference to the encoding format of the output data file comprising the digital data in order to identify the one or more physical parameters to which the concerned processing rule applies.

The digital data generated by the simulator may be encoded in various ways. The digital data generated by the simulator are encoded using an encoding format that enables to identify each value (or absence of value) for each physical parameter for each timestamp during the simulated surgical process. The digital data generated by the simulator may then be stored in an output data file according to the selected encoding format. For example a data table is used as encoding format in which one column correspond to a given physical parameter and a line corresponds to a given timestamp at which the physical parameter was determined. A cell of the data table corresponding to a given column and a given timestamp may further include one or more values (separated by a given separator character). For example, the cell may include a vector representing a displacement, a force in two or more dimensions with a value for each dimension.

In the example of FIG. 5A one activity detection rule is defined. The type of rule is indicated by a first data field 500 including a tag <activities_rules> identifying the type of rule, here activity detection rules. The semantic data associated with the activity detection rule is defined by the data field 501. In this example the activity is defined by two verbs "Catch" and "Drop" corresponding to two possible actions. One or more attribute fields may be used to define attributes of the concerned activity. In the example of FIG. 5A, the attributes include: a target ("Block1") to which the activity may be applied, a surgical tool ("Grasper"), an actor ("Surgeon") performing the activity, an effector ("left-hand") performing the activity, etc. In this example, the activity corresponds to an action of grabbing (or dropping) Block 1 with the Grasper tool manipulated by the surgeon's left hand.

One or more conditions may be defined for each activity rule. Each condition may be defined by one or more condition elements, whereby a condition element defines a logical test to be applied to a given data type. All the condition elements (<cond_element>) of a condition must be checked and fulfilled for detection of a given activity. The data type to which the logical test applies may be identified by an identifier referring to the encoding format of an output data file encoding the digital data outputted by the simulator. The logical test may be defined by a value to which the data having the given data type will be compared to.

In the example of FIG. 5A, three condition elements 506A, 506B, 506C are defined. Those condition elements are tested at a timestamp t. In a condition element, the tag <id> identifies a data field (e.g. a cell or value of a cell in the data table) containing the information to be checked in the digital data generated by the simulator. The data field may be identified by reference to the encoding format used by the simulator, e.g. by column / value identifiers identifying data field in the output data file of the simulator. In a condition element, the data field identified by the tag <value> specifies a value for applying the condition. Different type of condition elements may be used:
- a condition element that specifies that at least one of the data field on a list of data fields having the designated data type must have the value specified by the tag <value>;
- a condition element that specifies that all the data field in a list of data fields having the designated data type must have the value designated by the tag <value>;
- a condition element that specifies that exactly N data fields among a list of data fields having the designated data type must have the value designated by the tag <value>.

Back to the example of FIG. 5A, the first condition element 506A defines a value "1" and a data type identifier "14-5", meaning that that the concerned information (i.e. the concerned data field) is the 5th value contained in the cell to be found in the 14^{th} column of data table of the digital data file generated by the simulator. Similarly the second condition element 506B defines a value "0" and data field "15-5". The third condition element 506XC defines a value "0" and data field "15-12".

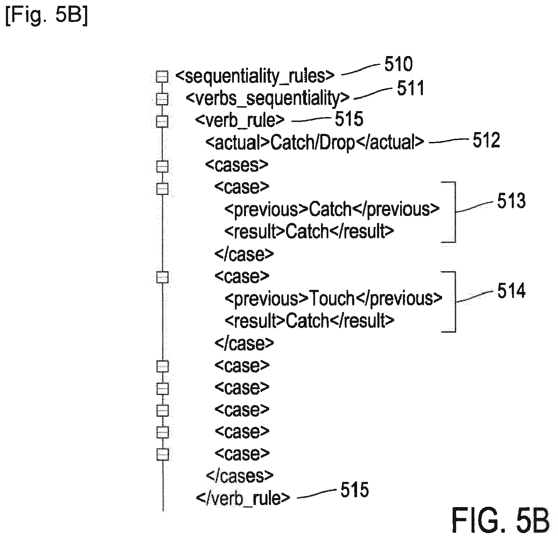

However, the information provided by the simulator alone is not always sufficient to differentiate all activities. In FIG. 5A, the information does not allow us to differentiate between the action of "Catch" or "Drop", hence the fact that the rule presented offers both possibilities. To differentiate between the two actions, it is necessary to take into account contextual information from the past, that is actions identified in the past. This is achieved through the sequentiality rules and illustrated by FIG. 5B. A tag 510 (<sequentiality_rules>) identifies the type of rule. A tag (<verbs_sequentiality>) 511 identifies the granularity level, here the activity level (also referred to as the verb level). In this simplified example, if the semantic data defining the different actions to be distinguished is "Catch/drop" (tag <actual>) 512, there are 7 cases (identified by the tag <case>), where the two first cases 513, 514have been deployed, that according to the action identified for the previous instant (<previous>) determine the correct current action (<result>). In particular, according to the first case 513, if the previously detected action is "Catch" then the current action will be "Catch". According to the second case 514, if the previously action is "Touch" then the current action will be also "Catch".

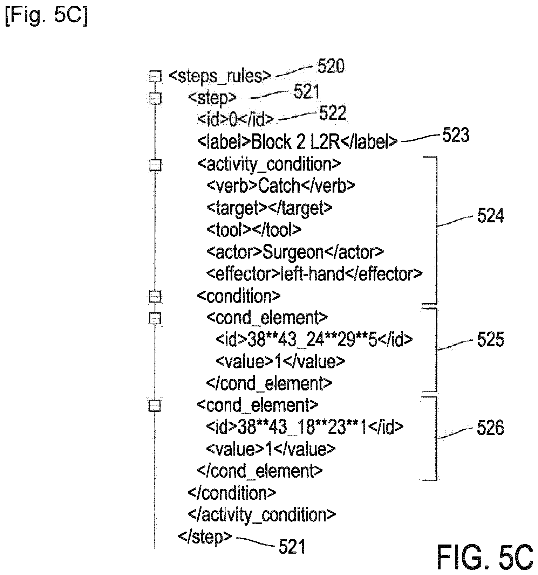

In the example of FIG. 5C one step detection rule is defined. To determine the beginning of the surgical process step, both the information about the one or more detected activities (<activity_condition>) as well as the digital data from the simulator (<condition>) are checked. Like for the activity level, all the condition elements (<cond_element>) of a condition must be checked and fulfilled for detection of a given surgical process step. In the case of surgical process steps, only the beginning of a surgical process step need to be detected, since the beginning of a surgical process step marks the end of the previous surgical process step.

Back to the example of FIG. 5C, the granularity level is indicated by a tag 520 (<steps_rules>). The definition of the step detection rule starts and ends with a tag <step> 521. The semantic data associated with the step detection rule is defined by the data field 523 introduced with the tag <label>. The identification of the surgical process step is defined by the data field 522 introduced with the tag <id>, here a step identifier (step number).

In the example of FIG. 5C, the one or more activity conditions may be defined (<activity_condition>) by one or more attributes of a surgical process activity:
- a verb defining an activity, the data field being introduced by the tag <verb> and defined by semantic data ("Catch");
- a target to which the activity may be applied, the data field being introduced by the tag <target> and defined by semantic data (in the example the data field is an empty field ""),
- a surgical tool used to perform the action, the data field being introduced by the tag <tool> and defined by semantic data (in the example the data field is an empty field ""),
- an actor performing the activity, the data field being introduced by the tag <actor> and defined by semantic data ("Surgeon");
- an effector performing the activity, the data field being introduced by the tag <effector> and defined by semantic data ("left-hand").

In this example, when the data field of the value associated with an activity condition is empty, it means that the data field of the tested surgical process activity may have any value.

In the example of FIG. 5C, two condition elements 525 and 526 are defined. The first condition element represents a complex case where several data fields of the digital data from the simulator are considered. For the first condition element 525, the data fields of the digital data are identified by the data field (38**43_24**29**5) associated with the tag <id>, which means columns 38 to 43 (6 columns) and 24th to 29th values in the cells of columns 38 to 43 of the table encoding the digital data from the simulator are tested and that among these 36 data fields (6 columns * 6 cell values), 5 must have the value ("1") defined by the tag <value> for the first condition element to be fulfilled. For the second condition element, the data fields of the digital data are identified by the data field (38**43_18**23**1) associated with the tag <id>, which means columns 38 to 43 (6 columns) and 18th to 23rd values in the cells of columns 38 to 43of the table encoding the digital data from the simulator are tested and that among these 36 data fields (6 columns * 6 cell values), 1 must have the value ("1") defined by the tag <value> for the second condition element to be fulfilled.

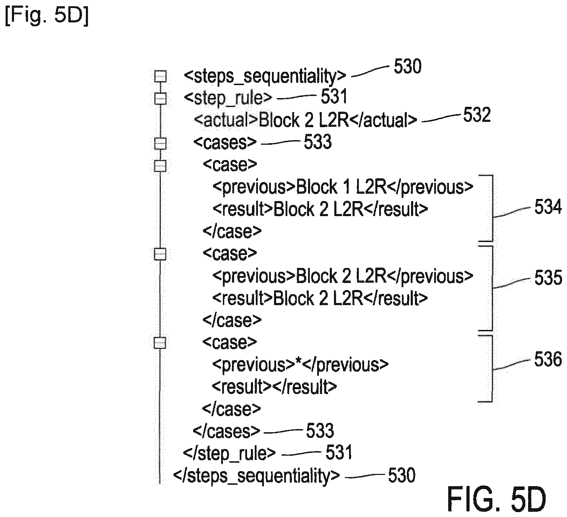

Step sequentiality rules and illustrated by FIG. 5D. A tag 530 (<step_sequentiality>) identifies the type of rule. A tag (<step_rules>) 531 identifies the granularity level, here the step level. A tag (<actual>) 532 identifies the semantic data ("Block 2 L2R") associated with the current step. This surgical process step corresponds to the transfer of the second block ("Block 2") from left to right. There are 3 different possible cases, introduced by the tag (<cases>) 533, in which this surgical process step may be detected:
- in case 534, the previous surgical process step, introduced by the tag <previous> 533A, was the transfer of the first block from left to right (Block 1 L2R), in this case the semantic data associated with the current step is "Block 2 L2R", introduced by tag <result>; This is the logical sequentiality;
- in case 535, the previous surgical process step, introduced by the tag <previous>, was already "Block 2 L2R", so the semantic data associated with the current step is also "Block 2 L2R", introduced by tag <result>; This case may occur if the conditions for determining a surgical process step may occur several times during the concerned surgical process step;
- in case 536, the previous surgical process step is neither "Block 1 L2R" nor "Block 2 L2R" introduced by the tag <previous> (field value is "*"), so the semantic data for the current surgical process step introduced by tag <result> is empty (no semantic data or label); in practice, if this happens, it is because the rules are poorly defined or because the simulator information is insufficient.

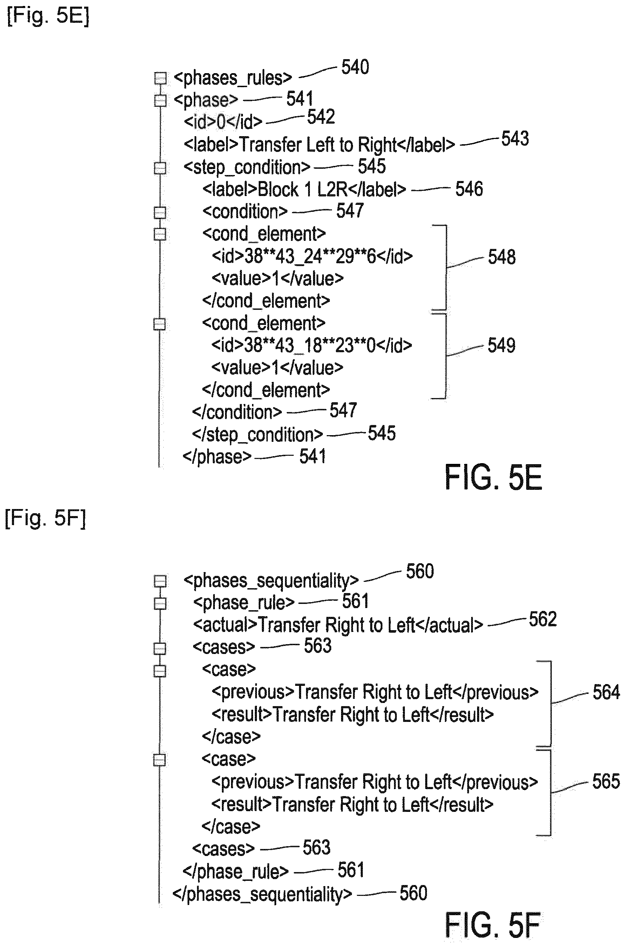

In the example of FIG. 5E, the granularity level is indicated by a tag 540 (<phases_rules>). The definition of the phase detection rule starts and ends with a tag <phase> 541. The semantic data "Transfer Right To Left" associated with the phase detection rule is defined by the data field 543 introduced with the tag <label>. The identification of the surgical process phase is defined by a data field introduced with the tag <id> 542, here a phase identifier (phase number).

As will be illustrated by FIG. 5E, to determine the beginning of the phases, we use the step information for previously detected steps (tag <step_condition> 545) as well as the digital data from the simulator to determine additional conditions (tag <condition> 547). As for the activities and steps, all the condition elements (tag <cond_element> 548, 549) of a given condition 547 must be fulfilled in order for the condition itself to be fulfilled.

Phase sequentiality rules and illustrated by FIG. 5F. A tag 560 (<phase_sequentiality>) identifies the type of rule, here a phase sequentiality rule. A tag (<phase_rule>) 561 identifies the granularity level, here the phase level. A tag (<actual>) 562 identifies the semantic data ("Block 2 L2R") associated with the current step. This surgical process phase corresponds to the transfer of 6 blocks from right to left. There are 2 different possible cases 564, 565, introduced by the tag (<cases>) 563, in which this surgical process phase may be detected:
- in case 564, the previous phase (introduced by the tag (<previous>)) was the transfer of the 6 blocks from left to right ("Transfer Left To Right"), in this case the semantic data for the current phase is "Transfer Right To Left"; this is the logical sequentiality.
- in case 565, the previous phase (introduced by the tag (<previous>)) was already Transfer Right To Left, so the semantic data associated with the current phase is also "Transfer Right To Left"; this case may occur if the conditions for determining a phase may occur several times during said phase.

Unlike the example for the sequentiality rules of the steps (FIG. 5D), in the example of FIG. 5F, there are no cases for other phases, since in the example used here there are only 2 phases in the surgical process. However, a case similar to case 536 in the case of FIG. 5D illustrating the sequentiality rules of the steps could be foreseen, where no previous phase is recognized, introduced by the tag <previous> (field value is "*"), so that the semantic data for the current phase introduced by tag <result> is empty (no semantic data or label); in practice, if this happens, it is because the rules are poorly defined or because the simulator information is insufficient.

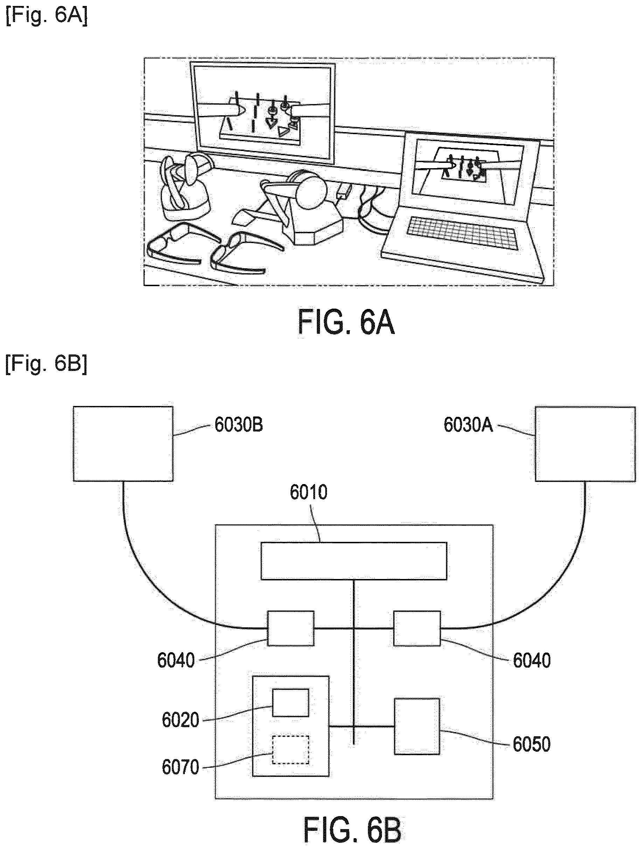

A set-up for implementing a method for converting (i.e. transcribing) a user-driven virtual surgical process performed using a simulator into a temporal sequence of semantic data describing a surgical process at a semantic level will now be described by reference to FIG. 6A.

The set-up may be a home-made surgical simulator developed at the department of mechanical engineering at the University of Tokyo [18]. The set-up includes:
- A core laptop (i7-700HQ, 16Go RAM, GTX 1070);
- A 3D rendering setup: 3D screen (24 inches, 144Hz) and 3D glasses;
- Two haptic interfaces.

The core laptop operates and implements the 3D rendering setup and the two haptic interfaces. A user can interact with the simulator by using the 3R rendering setup, which simulates a virtual surgical scene, and the haptic interfaces, which allow the user inputting control commands. For each surgical process simulation, the simulator provides video and simulator information, both synchronized at 30Hz.

For completeness, FIG. 6B is a schematic diagram showing components of a device (6000) configured to implement any method described herein.

The device (6000) may be implemented as a single hardware device, for example in the form of a desktop personal computer (PC), a laptop, a personal digital assistant (PDA), a smart phone, a server, a console or may be implemented on separate interconnected hardware devices interconnected by one or more communication links, with wired and/or wireless segments. The device (6000) may for example be in communication with one or more cloud computing systems, one or more remote servers or devices to implement the functions described herein for the concerned device. The device (6000) may also be implemented itself as a cloud computing system.

As represented schematically on **FIG. 6B**, the device (6000) may include at least one processor (6010) configured to access to at least one memory (6020) including a computer program code (6070). The computer program code may include instructions configured to cause the device (6000) to perform one or more or all steps of one or more methods described herein.

The processor (6010) may thus be configured to store, read, load, interpret, execute and/or otherwise process the computer program code (6070) stored in the memory (6020) such that, when the instructions encoded in the computer program code are executed by the at least one processor (6010), the device (6000) performs one or more steps of one or more method described herein.

The processor (6010) may be any suitable microprocessor, microcontroller, integrated circuit, or central processing unit (CPU) including at least one hardware-based processor or processing core.

The memory (6020) may include a random access memory (RAM), cache memory, non-volatile memory, backup memory (e.g., programmable or flash memories), read-only memory (ROM), a hard disk drive (HDD), a solid state drive (SSD) or any combination thereof. The ROM of the memory (6020) may be configured to store, amongst other things, an operating system of the device (6000) and / or one or more computer program code of one or more software applications. The RAM of the memory 6020 may be used by the processor (6010) for the temporary storage of data.

The device (6000) may further include one or more communication interfaces (6040) (e.g. network interfaces for access to a wired / wireless network, including Ethernet interface, WIFI interface, USB interfaces etc) configured to communicate via network and / or to one or more peripheral devices. The device (6000) may include other associated hardware such as user interfaces such as a 2D visual rendering interface (2030A), in our case containing here a 3D screen and 3D glasses, or haptic interfaces (2030B), or any other interfaces (e.g. keyboard, mice, display screen, etc) connected via appropriate communication interface(s) (6040) with the processor. The device (6000) may further include a media drive (6050) for reading an external computer-readable storage medium. The processor (6010) is connected to each of the other components in order to control operation thereof.

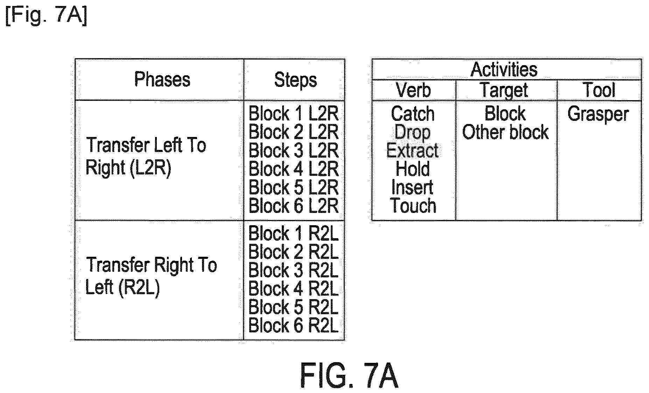

An example of an identification of semantic data at different granularities levels for a user-driven virtual surgical process performed using a simulator is illustrated in FIG. 7A.

The chosen procedure for this example is a peg transfer, and consists of transferring 6 blocks from the left to the right and the reverse. For this work, two surgical process phases, 12 surgical steps, 6 surgical process activity verbs, 2 object targets, and one object tool are identified. Each phase corresponds to one transferring direction. Each surgical step (6 surgical steps by surgical phase) corresponds to the transfer of one block in one direction, e.g. "Block1 L2R" corresponds to the transfer of the first block from the left to the right. For the activities, two targets: "block" and "other block" are differentiated. The "block" corresponds to the block concerned by the current step. By way of example, during the surgical step,"Block2 L2R" the "block" corresponds to the second block. The "other block" is an additional target used to differentiate when the user interacts with another block.

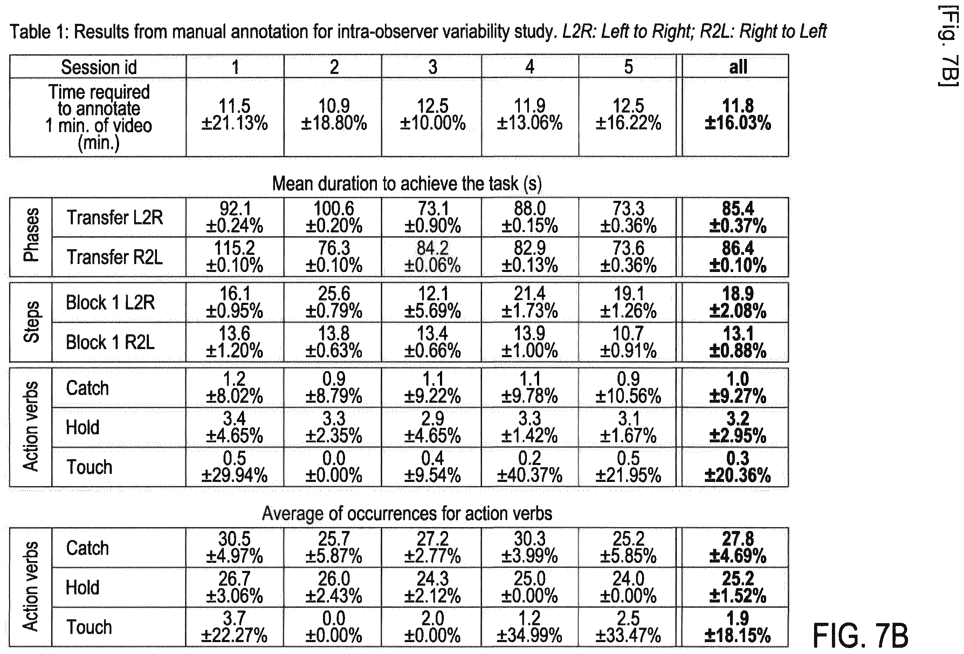

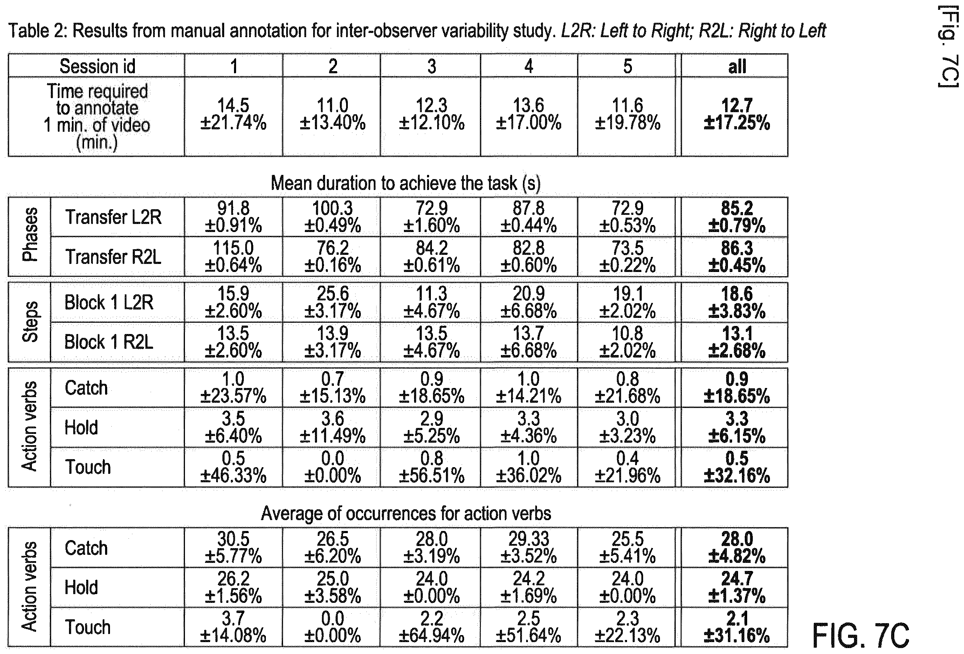

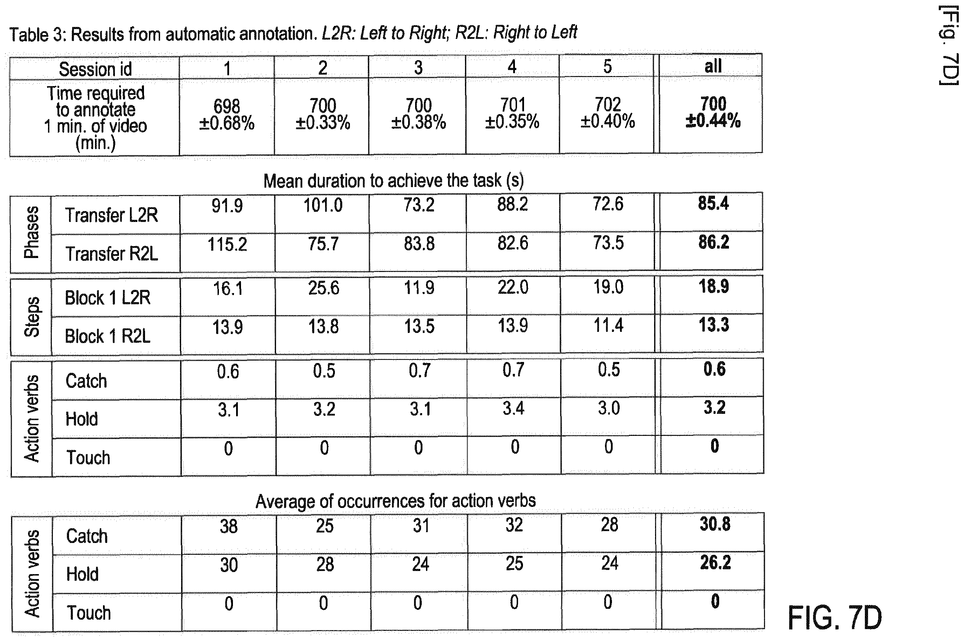

A comparison of the result of manual annotation a surgical process video of the surgical process of peg transfer described in FIG. 7A and automatic annotation of the same video performed by the proposed method will now be described by reference to FIGS. 7B, 7C and 7D.

Three summarizing tables are represented:
- Table 1 (FIG. 7B) represents the manual annotation results for an intra-observer study, that is, one user performs five times the manual annotation of the video.
- Table 2 (FIG. 7C) represents the manual annotation results for an inter-observer study, that is, five users perform one time the manual annotation of the video.
- Table 3 (FIG. 7D) represents the automatic annotation results obtained with the method according to the present disclosure and repeated several times.

In each table, for each annotation, whether manual or automatic, ("seq1", "seq2"...), the following data are entered:
- the time required to annotated one minute video,
- the duration of each phase, step (only the first step of each phase is represented for conciseness) and action verb annotation,
- the number of occurrence of each action verb.

The main results are as follows. For the manual annotation intra-observer study, the mean duration of the annotation of 1 minute of video is 11.8 minutes with a relative standard deviation (RSD) of 16.03%. The total number of annotation mistakes is 18. For the manual annotation inter-observer study, the mean duration of the annotation of 1 minute of video is 12.7 minutes with a relative standard deviation (RSD) of 17.25%. The total number of annotation mistakes is 47. For the automatic annotation study, the mean duration of the annotation of 1 minute of video is 700 millisecond with a relative standard deviation (RSD) of 0.44%. Those results outperform the results obtained during the manual annotation studies, in terms of annotation speed (the manual annotation is more than 700 times more time-consuming than automatic annotation) and variation. In particular, except for the time required to perform the annotation, there is no variation with the automatic annotation method. Regarding action verb variability with respect to durations and occurrences, this variability could be caused by the user own interpretation of the beginning and the end of a surgical process activity, or the difficulty difficult for the observers to start or stop an activity at the exact transition frame due to the annotation software inability to navigate frame by frame on the video.

The automatic annotation has thus multiple advantages compared to manual ones. It is faster, it is not subject of variability. If all activities could be interpreted by the flags derived from the simulator information 1001, it does not introduce annotation mistakes.

More widely than for the generation of surgical process models, the method according to the present disclosure could be used to provide data for the detection and classification of neural networks. The possibility to perform the annotation online could be also used to provide useful pedagogical guidance for trainees when performing the task and not afterward.

It should be appreciated by those skilled in the art that any block diagrams herein represent conceptual views of illustrative circuitry embodying the principles of methods, systems, devices, apparatus and computer program according to the present disclosure. Similarly, it will be appreciated that any flow charts, flow diagrams, state transition diagrams, pseudo code, and the like represent various processes which may be substantially represented in computer readable medium and executed by a computer or processor, whether or not such computer or processor is explicitly shown.

A further embodiment is a computer readable storage medium comprising computer program instructions for causing an apparatus to perform one or more steps of one or more of the methods described herein. The computer readable storage medium having computer readable program instructions embodied therein, which when being loaded on a computer, one or more processors and / or a programmable hardware component in an apparatus, cause the apparatus to perform one or more steps of one or more of the methods described herein. A further embodiment may be a computer program product comprising such a computer readable storage medium. The computer readable storage medium may be non-transitory.

A computer storage medium may be any physical media that can be read, written or more generally accessed by a computer. Embodiments of a computer-readable medium includes, but are not limited to, both computer storage media and communication media including any medium that facilitates transfer of a computer program from one place to another. Specifically, computer readable program instructions to perform embodiments described herein may be stored, temporarily or permanently, in whole or in part, on a non-transitory computer readable medium of a local or remote storage device including one or more storage media.

Examples of computer storage media include, but are not limited to, a flash drive or other flash memory devices (e.g. memory keys, memory sticks, USB key drive), CD-ROM or other optical storage, DVD, magnetic disk storage or other magnetic storage devices, solid state memory, memory chip, RAM, ROM, EEPROM, smart cards, a relational database management system, a traditional database, or any other suitable medium from that can be used to carry or store computer program instructions in the form of instructions or data structures which can be read by a computer processor. Also, various forms of computer-readable medium may be used to transmit or carry instructions to a computer, including a router, gateway, server, or other transmission device, wired (coaxial cable, fiber, twisted pair, DSL cable) or wireless (infrared, radio, cellular, microwave). The computer program instructions may include code from any computer-programming language, including, but not limited to, assembly, C, C++, Basic, SQL, MySQL, HTML, PHP, Python, R, Julia, Java, Javascript, etc.

A further embodiment relates to an apparatus or device comprising means configured to perform one or more steps of one or more methods described herein. The "means configured to perform" a given function shall be understood as functional block(s) comprising circuitry that is adapted for performing or configured to perform the given function. Moreover, any entity described herein as "means", may correspond to or be implemented as "one or more modules", "one or more devices", "one or more units", e.g "one or more processing units", etc. Means for performing one or more functions may also comprises one or more processors and one or more memories (e.g. in a system or apparatus) for storing computer program instructions configured to, when executed by at least one processor, cause the performance (e.g. by the system or apparatus) of the one or more functions.

When provided by a processor, the functions may be provided by a single dedicated processor, by a single shared processor, or by a plurality of individual processors, some of which may be shared. Moreover, explicit use of the term "processor" may implicitly include, digital signal processor (DSP) hardware, network processor, application specific integrated circuit (ASIC), field programmable gate array (FPGA), etc. Other hardware, conventional or custom, may also be included. Their function may be carried out through the operation of program logic, through dedicated logic, through the interaction of program control and dedicated logic, or even manually, the particular technique being selectable by the implementer as more specifically understood from the context.

A memory may include a random access memory (RAM), cache memory, non-volatile memory, backup memory (e.g., programmable or flash memories), read-only memory (ROM), a hard disk drive (HDD), a solid state drive (SSD) or any combination thereof. The ROM may be configured to store, amongst other things, an operating system of the system / apparatus and / or computer program instructions. The RAM may be used by the processor(s) for the temporary storage of data.

### List of References

[1] F. Lalys and P. Jannin. Surgical process modelling: a review. International Journal of Computer Assisted Radiology and Surgery, 9(3):495-511, September 2013.
[2] F. Despinoy, D. Bouget, G. Forestier, C. Penet, N. Zemiti, P. Poignet, and P. Jannin. Unsupervised trajectory segmentation for surgical gesture recognition in robotic training. IEEE Transactions on Biomedical Engineering, 63(6):1280-1291, 2015.
[3] A. Huaulme, K. Harada, G. Forestier, M. Mitsuishi, and P. Jannin. Sequential surgical signatures in micro-suturing task. International Journal of Computer Assisted Radiology and Surgery, 13(9):1-10, May 2018. ISSN 1861-6410, 1861-6429. doi: 10.1007/s11548-018-1775-x.
[4] Germain Forestier, Laurent Riffaud, François Petitjean, Pierre-Louis Henaux, and Pierre Jannin. Surgical skills: Can learning curves be computed from recordings of surgical process activities? International Journal of Computer Assisted Radiology and Surgery, 13(5):629-636, May 2018. ISSN 1861-6410, 1861-6429. doi: 10.1007/s11548-018-1713-y.
[5] W. S. Sandberg, B. Daily, M. Egan, J. E. Stahl, J. M. Goldman, R. A. Wiklund, and D. Rattner. Deliberate Perioperative Systems Design Improves Operating Room Throughput:. Anesthesiology, 103(2):406-418, August 2005. ISSN 0003-3022. doi: 10.1097/00000542-200508000-00025
[6] B. Bhatia, T. Oates, Y. Xiao, and P. Hu. Real-time identification of operating room state from video. volume 2, pages 1761-1766, 2007.
[7] S.-Y. Ko, J. Kim, W.-J. Lee, and D.-S. Kwon. Surgery task model for intelligent interaction between surgeon and laparoscopic assistant robot. International Journal of Assitive Robotics and Mechatronics, 8(1):38-46, 2007.
[8] G. Quellec, M. Lamard, B. Cochener, and G. Cazuguel. Real-Time Task Recognition in Cataract Surgery Videos Using Adaptive Spatiotemporal Polynomials. IEEE Transactions on Medical Imaging, 34(4):877-887, April 2015. ISSN 0278-0062. doi: 10.1109/TMI.2014.2366726.
[9] T. Neumuth, R. Wiedemann, C. Foja, P. Meier, J. Schlomberg, D. Neumuth, and P. Wiedemann. Identification of surgeon\individual treatment profiles to support the provision of an optimum treatment service for cataract patients. Journal of Ocular Biology, Diseases, and Informatics, 3(2):73-83, June 2010.
[10] N. Padoy, T. Blum, S.-A. Ahmadi, H. Feussner, M.-O. Berger, and N. Navab. Statistical modeling and recognition of surgical workflow. Medical Image Analysis, 16(3):632-641, 2010.
[11] A. P. Twinanda, S. Shehata, D. Mutter, J. Marescaux, M. de Mathelin, and N. Padoy. EndoNet: A Deep Architecture for Recognition Tasks on Laparoscopic Videos. IEEE Transactions on Medical Imaging, 36(1):86-97, January 2017. ISSN 0278-0062. doi: 10.1109/TMI.2016.2593957.
[12] L. Bouarfa, P. P. Jonker, and J. Dankelman. Discovery of high-level tasks in the operating room. Journal of Biomedical Informatics, 44(3):455-462, June 2011.
[13] A. James, D. Vieira, B. Lo, A. Darzi, and G.-Z. Yang. Eye-Gaze Driven Surgical Workflow Segmentation. Medical Image Computing and Computer-Assisted Intervention MICCAI 2007, pages 110-117, October 2007.
[14] F. Lalys, D. Bouget, L. Riffaud, and P. Jannin. Automatic knowledge- based recognition of low-level tasks in ophthalmological procedures. International Journal of Computer Assisted Radiology and Surgery, 8(1):39-49, April 2012.
[15] Y. Gao, S. S. Vedula, C. E. Reiley, N. Ahmidi, B. Varadarajan, H. C. Lin, L. Tao, L. Zappella, B. Bejar, D. D. Yuh, C. C. G. Chen, R. Vidal, S. Khudanpur, and G. D. Hager. JHU-ISI Gesture and Skill Assessment Working Set (JIGSAWS): A Surgical process activity Dataset for Human Motion Modeling. Modeling and Monitoring of Computer Assisted Interventions (M2CAI) MICCAI Workshop, page 10, 2014.
[16] D. Sarikaya, J. J. Corso, and K. A. Guru. Detection and Localization of Robotic Tools in Robot-Assisted Surgery Videos Using Deep Neural Networks for Region Proposal and Detection. IEEE Transactions on Medical Imaging, 36(7):1542-1549, July 2017. ISSN 0278-0062. doi: 10.1109/TMI.2017.2665671.
[17] Odysseas Zisimopoulos, Evangello Flouty, Mark Stacey, Sam Muscroft, Petros Giataganas, Jean Nehme, Andre Chow, and Danail Stoyanov. Can surgical simulation be used to train detection and classification of neural networks? Healthcare Technology Letters, 4(5):216-222, September 2017. ISSN 2053-3713. doi: 10.1049/htl.2017.0064. URL https://www.ncbi.nlm.nih.gov/pmc/articles/PMC5683210/.
[18] Heredia Perez, S.A., Harada, K., and Mitsuishi, M. Haptic Assistance for Robotic Surgical Simulation. 27th Annual Congress of Japan Society of Computer Aided Surgery, 20(4):232-233, November 2018.

## Claims

1. Method for converting a user-driven virtual surgical process performed using a simulator into a temporal sequence of semantic data describing a surgical process at a semantic level, the method comprising:
- obtaining (100) from the simulator at least one temporal sequence of digital data describing at a physical level the virtual surgical process simulated by the simulator in a virtual space, wherein the digital data comprise interaction data representing interactions between objects in the virtual space and kinematic data related to object displacements in the virtual space;
- automatically converting (1020-1060; 1120-1180), based on at least one configuration file, said at least one temporal sequence of digital data into said temporal sequence of semantic data,
wherein the at least one configuration file comprises semantic data representative of one or more surgical process activities and a set of one or more activity detection rules respectively associated with one or more surgical process activities defined by the semantic data.

2. Method according to claim 1, wherein the automatically converting comprises
detecting (1020; 1120) at least one surgical process activity corresponding to at least one subset of the digital data complying with at least one activity detection rule associated with the at least one surgical process activity by applying (1120) the set of one or more activity detection rules to one or more subsets of the digital data, and
determining semantic data representative of at least one detected surgical process activity.

3. Method according to claim 2, wherein an activity detection rule defines one or more conditions to be met by a subset of the digital data, wherein detecting (1020; 1120) at least one surgical process activity comprises
- filtering (200A, 200B) the interaction data to obtain one or more sequences of flag values corresponding to respective timestamps, wherein a flag value indicates whether an interaction between two objects occurs at a given timestamp;
- applying (210A, 210B) to the one or more sequences of flag values one or more activity detection rules to detect (1120) one or more pseudo-activities corresponding each to timestamp of the virtual surgical process;
- detecting (220A, 220C) for a first timestamp at least one surgical process activity based on the one or more detected pseudo-activities and contextual information, wherein the contextual information comprises filtered flag values obtained for a timestamp equal or prior to the first timestamp and / or one or more surgical process activities detected for a timestamp prior to the first timestamp.

4. Method according to claim 2 or 3, wherein the at least one configuration file comprises a set of one or more activity sequentiality rules, wherein method further comprises detecting (1130) at least one sequence of surgical process activities by applying (1130) the set of one or more activity sequentiality rules to one or more subsets of detected surgical process activities.

5. Method according to any of claims 2 to 4, wherein the at least one configuration file further comprises semantic data representative of one or more surgical process steps and a set of one or more step detection rules respectively associated with one or more surgical process steps defined by the semantic data, wherein a surgical process step corresponds to a sequence of surgical process activities occurring between a starting point and an ending point,
the method further comprising
detecting (1040; 1140) at least one surgical process step corresponding to a subset of at least one detected surgical process activity complying with at least one step detection rule associated with the at least one surgical process step by applying (1140) the set of one or more step detection rules to one or more subsets of the digital data and / or one or more sequence of detected surgical process activities; and
determining (1140) semantic data representative of at least one detected surgical process step.

6. Method according to claim 5, wherein the at least one configuration file comprises a set of one or more step sequentiality rules, wherein method further comprises detecting (1150) at least one sequence of surgical process steps by applying (1150) the set of one or more step sequentiality rules to one or more subsets of detected surgical process steps.

7. Method according to claim 5 or 6, wherein the at least one configuration file further comprises semantic data representative of one or more surgical process phases and a set of one or more phase detection rules respectively associated with one or more surgical process phases defined by the semantic data, wherein a surgical process phase corresponds to a sequence of surgical process steps occurring between a starting point and an ending point,
the method further comprising
detecting (1060; 1160) at least one surgical process phase corresponding to a subset of one or more detected surgical process steps complying with at least one phase detection rule associated with the at least one surgical process phase by applying (1160) the set of one or more phase detection rules to one or more subsets of the digital data and / or to one or more sequence of detected surgical process steps; and
determining (1160) semantic data representative of at least one detected surgical process step.

8. Method according to claim 7, wherein the at least one configuration file comprises a set of one or more phase sequentiality rules, wherein method further comprises detecting (1170) at least one sequence of surgical process phases by applying (1170) the set of one or more phase sequentiality rules to one or more subsets of detected surgical process steps.

9. Method for automatically generating a surgical process model, said method comprising:
converting (300) a user-driven virtual surgical process performed using a simulator into semantic data describing a surgical process at a semantic level by applying a method according to any of the preceding claims, the semantic data describing one or more sequence of surgical process activities and / or one or more surgical process steps and / or one or more surgical process phases;
generating (300) the surgical process model based on the semantic data.

10. Method according to claim 9, comprising:
automatically annotating (310) video data issued from the simulator by using the semantic data to generated an annotated video, wherein the surgical process model includes the annotated video.

11. A computer readable medium comprising computer program instructions which when executed causes an apparatus to perform the steps of the method according to any of the claims 1 to 10.

12. A device comprising means for performing the steps of the method according to any of claims 1 to 10.
